(19) Europäisches Patentamt
European Patent Office
Office européen des brevets

(11) Numéro de publication: **0 209 511**
**B1**

# (12) FASCICULE DE BREVET EUROPÉEN

(45) Date de publication du fascicule du brevet: **06.12.89**

(51) Int. Cl.⁴: **C 07 D 471/04,** A 61 K 31/435 // (C07D471/04, 221:00, 209:00)

(21) Numéro de dépôt: **86870099.8**

(22) Date de dépôt: **02.07.86**

## (54) Chlorhydrates de chlorures de dérivés d'aminoalkyl-2 hydroxy-9 ellipticinium et compositions pharmaceutiques en contenant.

(30) Priorité: **04.07.85 FR 8510257**

(43) Date de publication de la demande: **21.01.87 Bulletin 87/4**

(45) Mention de la délivrance du brevet: **06.12.89 Bulletin 89/49**

(84) Etats contractants désignés: **AT BE CH DE FR GB IT LI LU NL SE**

(56) Documents cité:
**EP-A-0 009 445**
**EP-A-0 042 348**

(73) Titulaire: **SANOFI, 40, Avenue George V, F-75008 Paris (FR)**
Titulaire: **Établissement Public dit: CENTRE NATIONAL DE LA RECHERCHE SCIENTIFIQUE (CNRS), 15, Quai Anatole France, F-75007 Paris (FR)**

(72) Inventeur: **Gros, Pierre, Les Fontaines Rue des Mûriers, 18, F-34100 Montpellier (FR)**
Inventeur: **Crisafulli, Emilio, Viale San Gimignano, 12, I-20146 Milano (IT)**
Inventeur: **Mazue, Guy, Ferme Bosne, F-39230 Mantry (FR)**

(74) Mandataire: **Moncheny, Michel, c/o Cabinet Lavoix 2 Place d'Estienne d'Orves, F-75441 Paris Cedex 09 (FR)**

EP 0 209 511 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

## Description

La présente invention concerne les chlorhydrates des chlorures de dérivés d'aminoalkyl-2 hydroxy-9 ellipticinium, un procédé pour leur préparation ainsi que des compositions pharmaceutiques contenant lesdits composés en tant qu'ingrédients actifs.

On a décrit dans le brevet No. 4 310 667 des Etats Unis d'Amérique des sels quaternaires hydrosolubles d'hydroxy-9 ellipticines de formule:

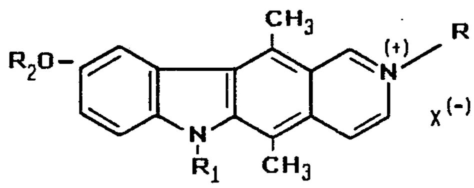

(I)

dans laquelle $R_1$ est l'hydrogène ou un groupe alkyle, $R_2$ est l'hydrogène, un groupe alkyle ou un groupe acyle, R est un groupe alkyle éventuellement substitué et $X^{(-)}$ est un anion de quaternisation convenable.

Les sels quaternaires hydrosolubles de formule (I) ci-dessus sont préparés par traitement de l'hydroxy-9 ellipticine correspondante avec l'halogénure $R\text{-}X_1$, $X_1$ représentant un atome d'halogène, et par transformation de l'halogénure d'ellipticinium ainsi obtenu, pratiquement insoluble dans l'eau, en sel hydrosoluble correspondant par une résine échangeuse d'ions.

Dans le brevet U.S. No. 4 310 667 cité ci-dessus tous les exemples relatifs aux halogénures quaternaires intermédiaires ne concernent que des bromures ou des iodures.

Parmi les sels quaternaires hydrosolubles du brevet ci-dessus les acétates sont indiqués comme étant préférés le composé de formule (I) dans laquelle $R_1$ et $R_2$ sont l'hydrogène, R représente méthyle et $X^{(-)}$ représente l'anion acétate est d'ailleurs utilisé en thérapeutique humaine pour le traitement de formes tumorales. Ce composé qui a reçu la Dénomination commune Internationale "acétate d'elliptinium" est actuellement disponible sur le marché sous forme de lyophilisat dosé à 60, 65 mg d'acétate d'elliptinium, lyophilisat destiné à être repris dans 10 ml d'eau pour préparations injectables.

D'autres composés, très actifs et plus hydrosolubles que l'acétate d'elliptinium ont été décrits dans le même brevet US, à savoir des composés de formule (I) ci-dessus dans laquelle $X^{(-)}$ est l'anion acétate, $R_1$ et $R_2$ sont l'hydrogène et R est un groupe éthyle substitué par un groupe diéthylamino ou pipéridino.

Cependant, ces acétates d'aminoalkyl-2 hydroxy-9 ellipticinium ainsi que leur préparation présentent plusieurs inconvénients tels qu'ils rendent ces composés pratiquement inutilisables sous forme de compositions pharmaceutiques. Par exemple les acétates d'aminoalkyl-2 hydroxy-9 ellipticinium en question se révèlent beaucoup plus instables que l'acétate d'elliptinium surtout en solution, ce qui se traduit dans la pratique par des opérations de lyophilisation et de mise en forme pharmaceutique d'une extrême difficulté.

En outre, la préparation des acétates des dérivés hydroxy-9 ellipticinium du brevet US cité précédemment nécessite lors d'une de ses étapes le passage sur une résine échangeuse d'anion.

Cette méthode présente cependant certains inconvénients. Par exemple, dans le cas de la préparation de l'acétate d'elliptinium, on a mentionné dans le brevet US en question, le passage transitoire à une solution métastable de dérivé d'ellipticinium, solution qu'il est nécessaire d'utiliser rapidement pour la suite des opérations.

Eu outre, cette méthode d'obtention de dérivés acétates donne fréquemment naissance à des composés secondaires particulièrement gênants lors de la préparation ultérieure de compositions pharmaceutiques.

En effet, on a trouvé que l'acétate formé à la suite de l'opération d'échange d'anion et notamment l'acétate des dérivés aminoalkyl-2 hydroxy-9 ellipticinium en particulier l'acétate de (diéthylamino-2 éthyl)-2 hydroxy-9 ellipticinium, se révèle souillé d'impuretés telles que des polymères ou des oligomères qu'il est nécessaire d'éliminer par une étape supplémentaire de purification avec notamment pour conséquence une perte de rendement en produit désiré.

On a maintenant trouvé, de manière surprenante, que les chlorhydrates des chlorures de dérivés d'aminoalkyl-2 hydroxy-9 ellipticinium présentent plusieurs avantages par rapport aux dérivés acétates antérieurs et notamment par rapport à l'acétate d'elliptinium.

Ainsi, on a pu mettre en évidence chez les chlorhydrates des chlorures de dérivés d'aminoalkyl-2 hydroxy-9 ellipticinium une très grande solubilité dans l'eau ainsi qu'une très bonne stabilité aussi bien à l'état solide qu'en solution. Par exemple, la solubilité dans l'eau du chlorhydrate du chlorure de (diéthylamino-2 éthyl)-2 hydroxy-9 ellipticinium est d'au moins 50 mg/ml.

En outre, la préparation de compositions pharmaceutiques contenant les chlorhydrates des chlorures de dérivés d'aminoalkyl-2 hydroxy-9 ellipticinium en question et notamment le chlorhydrate du chlorure de (diéthylamino-2 éthyl)-2 hydroxy-9 ellipticinium peut être réalisée très aisément.

Par conséquent, pour la préparation du principe actif destiné à être incorporé dans des spécialités pharmaceutiques on peut dissoudre 100 mg de chlorhydrate du chlorure de (diéthylamino-2 éthyl)-2 hydroxy-9 ellipticinium dans 2 ml d'eau en vue d'une lyophilisation et, ensuite, dissoudre 100 mg du lyophilisat ainsi obtenu dans

5 ou 10 ml d'eau pour préparations injectables.

Par contre, dans le cas de l'acétate d'elliptinium, on peut dissoudre 60 mg de ce sel dans 4 ml d'eau pour l'opération de lyophilisation. Cependant, la limite supérieure de solubilité du produit n'est que de 60 mg de lyophilisat dans 10 ml d'eau. En effet, à des concentrations plus importantes, la vitesse de dissolution de l'acétate d'elliptinium dans l'eau est trop lente compte tenu d'impératifs cliniques.

Il est donc possible de préparer des solutés de chlorhydrates de chlorures d'aminoalkyl-2 hydroxy-9 ellipticinium et notamment des solutés de chlorhydrate de chlorure de (diéthylamino-2 éthyl)-2 hydroxy-9 ellipticinium en concentration supérieure à celle de l'acétate d'elliptinium ce qui, associé à une toxicité moindre du produit, permet de mieux programmer le traitement du malade.

En outre, par rapport aux produits du brevet US No. 4 310 667, les chlorhydrates des chlorures ont l'avantage de ne pas comporter d'échange d'anion dans leur préparation, mais uniquement une simple salification avec de l'acide chlorhydrique, ce qui se traduira, sur le plan industriel, par une simplification de procédé et sur le plan de la pureté par une absence de produits secondaires indésirables.

On a également trouvé que lesdits chlorhydrates des chlorures de dérivés d'aminoalkyl-2 hydroxy-9 ellipticinium sont plus actifs que l'acétate d'elliptinium et, au moins aussi actifs que les composés correspondants portant l'anion acétate.

On a finalement trouvé que lesdits chlorhydrates des chlorures de dérivés d'aminoalkyl-2 hydroxy-9 ellipticinium sont moins toxiques et présentent moins d'effets secondaires que l'acétate d'elliptinium.

Ainsi, selon un de ses aspects, la présente invention a pour objet le chlorhydrate du chlorure d'un dérivé aminoalkyl-2 ellipticinium caractérisé par la formule suivante:

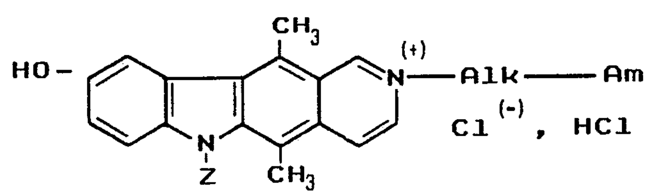

(II)

dans laquelle Z représente l'hydrogène ou un groupe alkyle inférieur, Alk représente un groupe alkylène inférieur et Am représente un groupe di(alkyle inférieur)amino, pyrrolidino ou pipéridino.

Le groupe "alkyle inférieur", désigne le radical monovalent d'un hydrocarbure aliphatique saturé contenant jusqu'à 4 atomes de carbone, choisi parmi les groupes méthyle, éthyle, propyle, isopropyle et n-butyle.

Le groupe "alkylène inférieur", désigne le radical bivalent non géminal d'un hydrocarbure aliphatique saturé contenant de 2 à 4 atomes de carbone, choisi parmi les groupes éthylène-1,2, propylène-1,2, propylène-1,3, et butylène-1,4.

Le groupe di(alkyle inférieur)amino représenté par Am peut être symétrique, comme dans le cas d'un radical diméthylamino, diéthylamino ou diisopropylamino, ou asymétrique, comme dans le cas d'un radical méthyléthylamino, méthylpropylamino ou éthylpropylamino.

Les dérivés aminoalkyl-2 ellipticinium de formule (II) dans laquelle-Alk-Am représente un radical diéthylaminoéthyle ou pipéridinoéthyle constituent une classe préférée de composés de l'invention.

En outre, le chlorhydrate du chlorure de (pipéridino-2 éthyl)-2 hydroxy-9 ellipticinium et plus particulièrement le chlorhydrate du chlorure de (diéthylamino-2 éthyl)-2 hydroxy-9 ellipticinium constituent des composés préférés de l'invention.

Selon un autre de ses aspects, la présente invention a pour objet un procédé pour la préparation des composés de formule II ci-dessus, caractérisé en ce que l'on traite une hydroxy-9 ellipticine de formule:

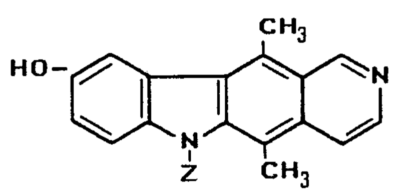

(III)

dans laquelle Z est tel que défini ci-dessus, avec une chloroalkylamine de formule:

Cl-Alk-Am (IV)

dans laquelle Alk et Am sont tels que définis ci-dessus, dans un solvant organique inerte à une température comprise entre la température ambiante et 140°C puis l'on transforme le chlorure quaternaire ainsi obtenu de formule:

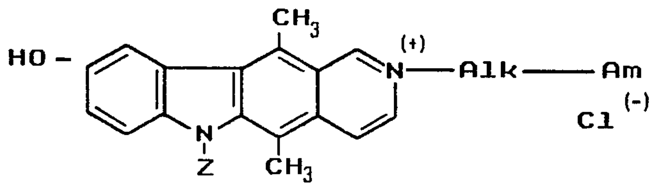

(V)

dans laquelle Z, Alk et Am sont tels que définis ci-dessus, en son chlorhydrate par traitement avec l'acide chlorhydrique.

La réaction des composés (III) avec les composés (IV) est conduite selon les méthodes connues en littérature pour la préparation de composés d'ammonium quaternaire.

De préférence, le solvant utilisé est un solvant aprotique polaire tel que le diméthylformamide, le diméthylacétamide ou le diméthylsulfoxyde, mais d'autres solvants, inertes dans les conditions de réaction, peuvent être utilisés.

Le temps de réaction dépend de la température; en général, à une température de 90 a 130°C la réaction est complète après 2 - 4 heures de chauffage et le produit (V) ainsi obtenu est isolé selon les modes opératoires conventionnels, par exemple par précipitation avec un solvant approprié tel que l'éther diéthylique.

Les composés de formule (V) ci-dessus sont nouveaux.

La transformation des composés (V) en chlorhydrates de la présente invention est effectuée selon les méthodes bien connues de salification.

De préférence, la salification du composé (V) est effectuée avec une solution hydroalcoolique d'acide chlorhydrique et le chlorhydrate est isolé selon les techniques usuelles.

Les composés de formule (II) ci-dessus se sont montrés beaucoup plus actifs et moins toxiques que l'acétate d'elliptinium et leur activité antitumorale s'est révélée au moins égale à celle des acétates correspondants dans tous les tests d'activité antitumorale effectués.

Par exemple, on a testé l'activité antitumorale des composés de l'invention vis-à-vis de la leucémie murine L1210.

A cet effet, on administre par voie intrapéritonéale à des souris mâles CDF1 réparties en groupes de 10 animaux, un inoculum tumoral à raison de $1.10^5$ cellules de leucémie L1210 lignée LEA03B06 dans 0,1 ml d'eau distillée par souris.

Après 24h, on injecte par voie intrapéritonéale à une moitié des groupes de souris, une dose unique du composé à étudier et à l'autre moitié des groupes un même volume de solvant, cette dernière moitié servant de souris témoins.

Au 60ème jour parès l'inoculation, on détermine le nombre d'animaux survivants et on mesure l'activité par l'augmentation du temps de survie des animaux traités par rapport aux animaux témoins.

On exprime cette activité par la valeur:

$$\frac{T}{C} = \frac{\text{temps médian de survie des animaux traités}}{\text{temps médian de survie des animaux témoins}} \times 100$$

le temps médian de survie des animaux traités prenant en compte les animaux survivants à la fin du test.

Dans ce test, le composé de l'Exemple 1 (SR 95156 B) a montré une certaine activité à 10 mg/kg (1/10 survivant au 60ème jour) et une bonne activité à 20 mg/kg (3/10 survivants) et à 50 mg/kg (5/10 survivants).

Un autre composé représentatif de la présente invention, le SR 95157E (Exemple 2) s'est montré actif a 20 mg/kg (2/10 survivants) et à 50 mg/kg (5/10 survivants). A 50 mg/kg, la durée relative de survie T/C du SR 95156 B est de 375 % et celle du SR 95157 B de 386 %.

Dans le même test, aucun des animaux de contrôle et des animaux traités avec 50 mg/kg d'acétate d'elliptinium ou 200 mg/kg de fluoro-5 uracile n'a survécu. Administré à la dose de 10 mg/kg, l'acétate d'elliptinium induit d'ailleurs une durée relative de survie T/C de 154 % sans survivant.

Un test supplémentaire réalisé avec le SR 95156 B sous forme de composition pharmaceutique (100 mg de lyophilisat dissous dans 10 ml d'eau pour préparation injectable) a confirmé les résultats précédents. A la dose de 50 mg/kg la durée relative de survie T/C est de 439 % avec 5/10 souris survivantes.

En outre des tests de toxicité ont pu mettre en évidence une toxicité aiguë et une néphrotoxicité plus faibles des chlorhydrates de chlorure de l'invention par rapport à l'acétate d'elliptinium.

Ainsi, par voie intrapéritonéale chez la souris, le chlorhydrate du chlorure de (diéthylamino-2 éthyl)-2 hydroxy-9 ellipticinium (SR 95156 B) a révélé une toxicité aiguë $DL_{50}$ de 75 mg/kg alors que l'acétate d'elliptinium, dans les mêmes conditions, a montré une $DL_{50}$ de 10,2 mg/kg [Recent Results in Cancer Research, *74* pp. 107 - 123 (1980)]. De même, le Tableau suivant, qui reproduit les résultats obtenus lors de tests de néphrotoxicité effectués avec le chlorhydrate du chlorure de (diéthylamino-2 éthyl)-2 hydroxy-9 ellipticinium (SR 95156 B) en comparaison avec un dérivé acétate en l' occurrence l'acétate d'elliptinium, montre la supériorité du composé de l'invention sur le composé connu.

**Tableau**

*Néphrotoxicité comparée chez le rat après injection réitérée du composé à étudier.*

| Dose (5 mg/kg) | Poids de rein en valeur relative (g/100) | Urée (mM) | Créatinine µM | Histopathologie (tubulopathie proximale) |
|---|---|---|---|---|
| Acétate d'elliptinium | 0,87 | 6,5 | 91,2 | +++ |
| SR 95156 B | 0,70 | 3,8 | 60,4 | environ + |
| Témoins | 0,68 | 3,2 | 55,2 | rien |

+++ : importante
++ : moyenne
+ : faible

Les composés de l'invention peuvent être utilisés pour le traitement de formes tumorales sensibles aux dérivés de l'ellipticine.

A cet effet, on utilisera par exemple des doses de 50 à 500 mg/m$^2$ par cure de traitement, en général par traitement répétitif de 3 jours.

Ainsi, selon un autre de ses aspects, la présente invention a pour objet des compositions pharmaceutiques renfermant au moins un composé de formule (II) ci-dessus en tant qu'ingrédient actif.

Les compositions pharmaceutiques de l'invention peuvent être présentées sous des formes appropriées convenant à l'administration en thérapie humaine ou vétérinaire de préférence sous forme d'unité d'administration pour l'administration parentérale.

La quantité totale de principe actif peut varier de 50 à 500 mg par unité d'administration, ledit principe actif étant de préférence associé à des véhicules pharmaceutiques notamment à de l'eau distillée.

Généralement, on prépare des compositions selon l'invention en dissolvant dans l'eau le principe actif selon l'invention, en procédant à la lyophilisation de la solution et en introduisant le lyophilisat obtenu dans de l'eau pour préparation injectable ou du sérum physiologique.

La solution ainsi obtenue peut être introduite dans des flacons pour perfusion intraveineuse contenant par exemple du sérum glucosé.

La forme d'administration préférée sera constituée d'un flacon de 50 à 500 mg de principe actif selon l'invention destiné à être dissous dans 5 à 20 ml d'eau pour préparation injectable ou de sérum physiologique contenus dans une ampoule.

Les Exemples non limitatifs suivants illustrent l'invention.

**Exemple 1**

*Chlorhydrate du chlorure de (diéthylamino-2 éthyl)-2 hydroxy-9 ellipticinium.*

(a) *Chlorure de (diéthylamino-2 éthyl)-2 hydroxy-9 ellipticinium.*

On chauffe à 100°C une solution d'hydroxy-9 ellipticine (obtenue par neutralisation de 23,3 mmoles de son bromhydrate) dans 100 ml de diméthylformamide. On y ajoute alors progressivement et sous agitation, une solution de chloro-1 diéthylamino-2 éthane (obtenue par neutralisation de 58 mmoles de son chlorhydrate) dans 20 ml d'éther diéthylique tout en distillant l'éther au fur et à mesure de son introduction. Au bout de 3 heures à 100°C sous agitation, le produit de réaction commence à précipiter. Après refroidissement, on filtre le précipité. On le lave avec 10 ml de diméthylformamide et, ensuite, avec 2 fois 25 ml d'éther diéthylique puis on le sèche à 40°C sous pression réduite. On isole ainsi 6,9 g de chlorure de (diéthylamino-2 éthyl)-2 hydroxy-9 ellipticinium brut de couleur orange.

(b) *Chlorhydrate du chlorure de (diéthylamino-2 éthyl)-2 hydroxy-9 ellipticinium.*

On dissout 6,9 g de chlorure de (diéthylamino-2 éthyl)-2 hydroxy-9 ellipticinium dans une solution formée par 2,45 ml d'acide chlorhydrique 12N, 26 ml d'eau et 180 ml d'éthanol, solution préalablement portée au reflux.

On ajoute alors 0,25 g de charbon activé et on filtre la solution à chaud. Après refroidissement, on filtre le précipité, on le lave avec 20 ml d'éthanol absolu puis avec 2 fois 30 ml d'acétone. On le sèche ensuite à 50°C pendant 12 heures. On obtient ainsi 5,64 g de chlorhydrate du chlorure de (diéthylamino-2 éthyl)-2 hydroxy-9 ellipticinium pur de couleur rouge brique (SR 95156 B).

Pureté par CLHP (chromatographie liquide haute pression): 99,8 %

Les spectres IR (infra-rouge) et H-RMN (résonnance magnétique nucléaire du proton) confirment la structure du produit.

Le rendement global est de 56 % par rapport au bromhydrate d'hydroxy-9 ellipticine.

**Exemple 2**

*Chlorhydrate du chlorure de (pipéridino-2 éthyl)-2 hydroxy-9 ellipticinium.*

En opérant comme décrit dans l'Exemple 1 (a), on traite à 110 - 130°C pendant 5 heures, une solution d'hydroxy-9 ellipticine (obtenue par neutralisation de 44 mmoles de son bromhydrate) dans 480 ml de diméthylformamide, par une solution de chloro-1 pipéridino-2 éthane (obtenue par neutralisation de 110 mmoles de son chlorhydrate), on salifie alors une partie (15 g) du chlorure de (pipéridino-2 éthyl)-2 hydroxy-9 ellipticinium de couleur orange ainsi obtenu avec une solution de 5,1 ml d'acide chlorhydrique 12N, 260 ml d'eau et 240 ml d'éthanol comme décrit dans l'Exemple 1 (b). On obtient ainsi 12,3 g de chlorhydrate du chlorure de (pipéridino-2 éthyl)-2 hydroxy-9 ellipticinium pur de couleur orange (SR 95157 B).

Analyse élémentaire (ramenée au produit desséché):

| | | | | |
|---|---|---|---|---|
| Calculé | : | C % : 64,57 | H % : 6,55 | N % : 9,41 |
| Trouvé | : | 64,68 | 6,55 | 9,71 |

| | | |
|---|---|---|
| Chlorures | : | 16,56 % |
| Pureté CLHP | : | 99,4 % |

Les spectres IR et H-RMN confirment la structure du produit. Le rendement global est de 84 % par rapport au bromhydrate d'hydroxy-9 ellipticine.

**Exemple 3**

*Chlorhydrate du chlorure de (diéthylamino-2 éthyl)-2 hydroxy-9 ellipticinium.*

Le mode opératoire décrit dans l'Exemple 1 à été exactement répété à partir d'hydroxy-9 ellipticine obtenue par neutralisation de 300 g de son bromhydrate. Le produit final obtenu est identique au SR 95156 B de l'Exemple 1.

Rendement global par rapport au bromhydrate d'hydroxy-9 ellipticine: 68 %.

**Exemples 4 à 9**

En opérant comme décrit dans l'Exemple 1, par traitement d'hydroxy-9 ellipticine avec, respectivement, le chloro-1 diisopropylamino-2 éthane, le chloro-1 éthylméthylamino-2 éthane et le chloro-1 pyrrolidino-2 éthane, on obtient:

- le chlorhydrate du chlorure de (diisopropylamino-2 éthyl)-2 hydroxy-9 ellipticinium (Ex. 4);
- le chlorhydrate du chlorure d'(éthylméthylamino-2 éthyl)-2 hydroxy-9 ellipticinium (Ex. 5); et
- le chlorhydrate du chlorure de (pyrrolidino-2 éthyl)-2 hydroxy-9 ellipticinium (Ex. 6).

En remplaçant l'hydroxy-9 ellipticine par la méthyl-6 hydroxy-9 ellipticine et en opérant de la même façon, on obtient:

- le chlorhydrate du chlorure de (diisopropylamino-2 éthyl)-2 méthyl-6 hydroxy-9 ellipticinium (Ex. 7);
- le chlorhydrate du chlorure d'(éthylméthylamino-2 éthyl)-2 méthyl-6 hydroxy-9 ellipticinium (Ex. 8); et
- le chlorhydrate du chlorure de (pyrrolidino-2 éthyl)-2 méthyl-6 hydroxy-9 ellipticinium (Ex. 9).

**Exemples 10 à 13**

En faisant réagir le chloro-1 diéthylamino-2 éthane, avec respectivement la méthyl-6 hydroxy-9 ellipticine et l'éthyl-6 hydroxy-9 ellipticine comme décrit dans l'Exemple 1, on obtient:

- le chlorhydrate du chlorure de (diéthylamino-2 éthyl)-2 méthyl-6 hydroxy-9 ellipticinium (Ex. 10); et
- le chlorhydrate du chlorure de (diéthylamino-2 éthyl)-2 éthyl-6 hydroxy-9 ellipticinium (Ex. 11).

En remplaçant le chloro-1 diéthylamino-2 éthane par le chloro-1 pipéridino-2 éthane et en opérant de la même façon, on obtient:

- le chlorhydrate du chlorure de (pipéridino-2 éthyl)-2 méthyl-6 hydroxy-9 ellipticinium (Ex. 12); et
- le chlorhydrate du chlorure de (pipéridino-2 éthyl)-2 éthyl-6 hydroxy-9 ellipticinium (Ex. 13).

**Exemple 14**

On prépare une composition pharmaceutique injectable par dissolution de 100 mg du composé de l'Exemple 1 ou de l'Exemple 2 dans 2 ml d'eau, lyophilisation de la solution obtenue et redissolution du lyophilisat dans 10 ml d'eau pour préparation injectable.

**Revendications** pour les Etats Contractants: BE, CH, DE, FR, GB, IT, LI, LU, NL, SE

1. Chlorhydrate du chlorure d'aminoalkyl-2 hydroxy-9 ellipticinium de formule:

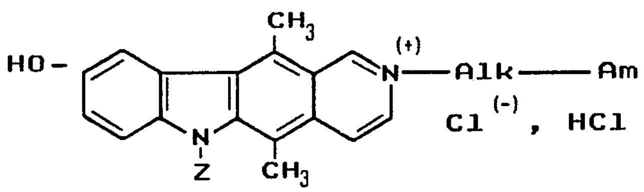

dans laquelle Z représente l'hydrogène ou un groupe alkyle inférieur, choisi parmi les groupes méthyle, éthyle, propyle, isopropyle et n-butyle, Alk représente un groupe alkylène inférieur choisi parmi les groupes éthylène-1,2, propylène-1,2, propylène-1,3 et butylène-1,4 et Am représente un groupe di(alkyle inférieur)amino choisi parmi les groupes diméthylamino, diéthylamino, diisopropylamino, méthyléthylamino, méthylpropylamino, éthylpropylamino, pyrrolidino et pipéridino.

2. Chlorhydrate du chlorure d'aminoalkyl-2 hydroxy-9 ellipticinium selon la Revendication 1 dans laquelle Alk représente un groupe éthylène et Am représente un groupe diéthylamino ou pipéridino.

3. Chlorhydrate du chlorure de (diéthylamino-2 éthyl)-2 hydroxy-9 ellipticinium.

4. Chlorhydrate du chlorure de (pipéridino-2 éthyl)-2 hydroxy-9 ellipticinium.

5. Chlorhydrate du chlorure d'aminoalkyl-2 hydroxy-9 ellipticinium selon la Revendication 1 pour leur application en tant que substances thérapeutiquement actives.

6. Chlorhydrate du chlorure de (diéthylamino-2 éthyl)-2 hydroxy-9 ellipticinium en tant qu'agent antitumoral.

7. Chlorhydrate du chlorure de (pipéridino-2 éthyl)-2 hydroxy-9 ellipticinium en tant qu'agent antitumoral.

8. Procédé pour la préparation des chlorhydrates de chlorure selon la Revendication 1, caractérisé en ce que l'on traite une hydroxy-9 ellipticine de formule:

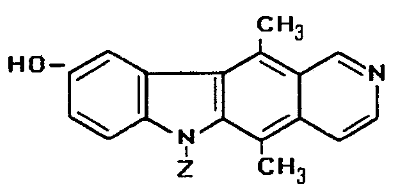

dans laquelle Z est tel que défini ci-dessus, avec une chloroalkylamine de formule:

Cl-Alk-Am

dans laquelle Alk et Am sont tels que définis ci-dessus, dans un solvant organique inerte à une température comprise entre la température ambiante et 140°C puis l'on transforme le chlorure quaternaire ainsi obtenu de formule:

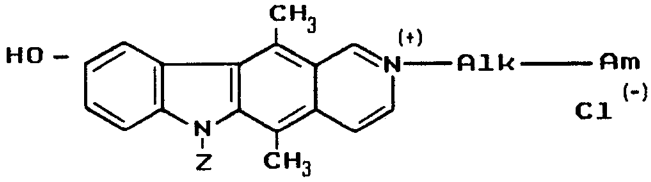

dans laquelle Z, Alk et Am sont tels que définis ci-dessus, en son chlorhydrate par traitement avec l'acide chlorhydrique.

9. Composition pharmaceutique caractérisée en ce qu'elle contient au moins un chlorhydrate de chlorure selon une des Revendications 1 à 4.

10. Composition pharmaceutique ayant une action antitumorale caractérisée en ce qu'elle contient au moins un chlorhydrate de chlorure selon une des Revendications 1 à 4 en association avec un véhicule pharmaceutique.

11. Composition pharmaceutique selon une des Revendications 9 et 10 caractérisée en ce qu'elle se présente sous forme d'unité d'administration pour l'administration parentérale.

**Revendications** pour l'Etat Contractant: AT

1. Procédé de préparation des chlorhydrates du chlorure d'aminoalkyl-2 hydroxy-9 ellipticinium de formule:

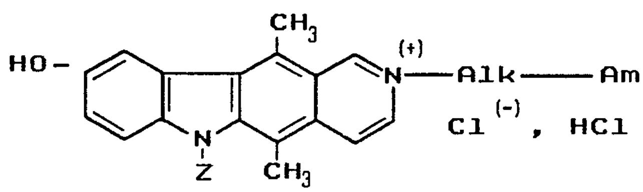

dans laquelle Z représente l'hydrogène ou un groupe alkyle inférieur, choisi parmi les groupes méthyle, éthyle, propyle, isopropyle et n-butyle, Alk représente un groupe alkylène inférieur choisi parmi les groupes éthylène-1,2, propylène-1,2, propylène-1,3, et butylène-1,4 et Am représente un groupe di(alkyle inférieur)amino choisi parmi les groupes diméthylamino, diéthylamino, diisopropylamino, méthyléthylamino, méthylpropylamino, éthylpropylamino, pyrrolidino et pipéridino, caractérisé en ce que l'on traite une hydroxy-9 ellipticine de formule:

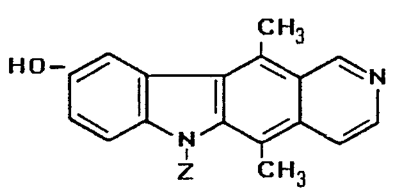

dans laquelle Z est tel que défini ci-dessus, avec une chloroalkylamine de formule:

Cl-Alk-Am

dans laquelle Alk et Am sont tels que définis ci-dessus, dans un solvant organique inerte à une température comprise entre la température ambiante et 140°C puis l'on transforme le chlorure quaternaire ainsi obtenu de formule:

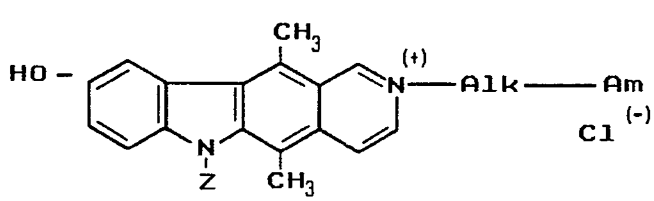

dans laquelle Z, Alk et Am sont tels que définis ci-dessus, en son chlorhydrate par traitement avec l'acide chlorhydrique.

2. *Procédé* selon la revendication 1, caractérisé en ce qu'on prépare les chlorhydrates du chlorure d'aminoalkyl-2 hydroxy-9 ellipticinium, dans lesquels Alk représente un groupe éthylène et Am représente un groupe diéthylamino ou pipéridino.

3. *Procédé* selon la revendication 1, caractérisé en ce qu'on prépare le chlorhydrate du chlorure de (diéthylamino-2 éthyl)-2 hydroxy-9 ellipticinium.

4. *Procédé* selon la revendication 1, caractérisé en ce qu'on prépare le chlorhydrate de chlorure de (pipéridino-2 éthyl)-2 hydroxy-9 ellipticinium.

5. *Procédé* de préparation d'une composition pharmaceutique ayant une action antitumorale, caractérisé en ce qu'on prépare au moins un chlorhydrate de chlorure selon l'une des revendications 1 à 4, en association avec un véhicule pharmaceutique.

**Patentansprüche** für die Vertragsstaaten: BE, CH, DE, FR, GB, IT, LI, LU, NL, SE

1. 2-Aminoalkyl-9-hydroxyellipticiniumchlorid-hydrochlorid der Formel (II):

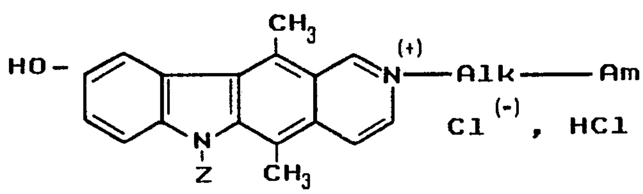

(II)

in der bedeuten:

Z Wasserstoff oder niedriges Alkyl, ausgewählt unter Methyl, Ethyl, Propyl, Isopropyl und n-Butyl,
Alk niedriges Alkylen, ausgewählt unter 1,2-Ethylen, 1,2-Propylen, 1,3-Propylen und 1,4-Butylen und
Am Di-(niedriges Alkyl)-amino, ausgewählt unter Dimethylamino, Diethylamino, Diisopropylamino, Methylethylamino, Methylpropylamino, Ethylpropylamino, Pyrrolidino und Piperidino.

2. 2-Aminoalkyl-9-hydroxyellipticiniumchlorid-hydrochlorid nach Anspruch 1 der Formel (II), in der Alk Ethylen und Am Diethylamino oder Piperidino ist.

3. 2-(2-Diethylaminoethyl)-9-hydroxyellipticiniumchloridhydrochlorid.

4. 2-(2-Piperidinoethyl)-9-hydroxyellipticiniumchloridhydrochlorid.

5. Verwendung der 2-Aminoalkyl-9-hydroxyellipticiniumchlorid-hydrochloride nach Anspruch 1 als therapeutisch aktive Substanzen.

6. 2-(2-Diethylaminoethyl)-9-hydroxyellipticiniumchlorid-hydrochlorid als Antitumormittel.

7. 2-(2-Piperidinoethyl)-9-hydroxyellipticiniumchlorid-hydrochlorid als Antitumormittel.

8. Verfahren zur Herstellung der Chlorid-hydrochloride nach Anspruch 1, dadurch gekennzeichnet, daß ein 9-Hydroxyellipticin der Formel (III):

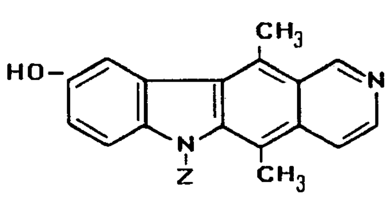

(III)

in der Z wie oben definiert ist, mit einem Chloralkylamin der Formel (IV):

Cl-Alk-Am (IV)

in der Alk und Am wie oben definiert sind,
in einem inerten organischen Lösungsmittel bei einer Temperatur von Raumtemperatur bis 140°C umgesetzt wird und

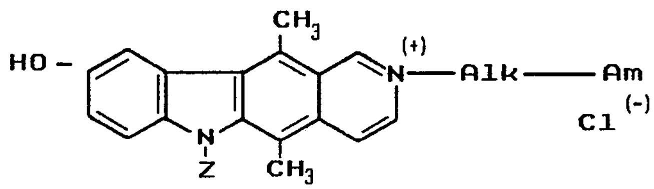

(V)

das so erhaltene quartäre Chlorid der Formel (V): in der Z, Alk und Am wie oben definiert sind, durch Behandlung mit Chlorwasserstoffsäure in das Hydrochlorid umgewandelt wird.

9. Pharmazeutische Zusammensetzung, gekennzeichnet durch den Gehalt an mindestens einem Chlorid-hydrochlorid nach einem der Ansprüche 1 bis 4.

10. Pharmazeutische Zusammensetzung mit Antitumorwirkung, gekennzeichnet durch den Gehalt an mindestens einem Chloridhydrochlorid nach einem der Ansprüche 1 bis 4 in Kombination mit einem pharmazeutischen Träger.

11. Pharmazeutische Zusammensetzung nach Anspruch 9 oder 10, gekennzeichnet durch eine Einzeldosisform für die parenterale Verabreichung.

**Patentansprüche** für den Vertragsstaat: AT

1. Verfahren zur Herstellung von 2-Aminoalkyl-9-hydroxyellipticiniumchlorid-hydrochloriden der Formel (II):

(II)

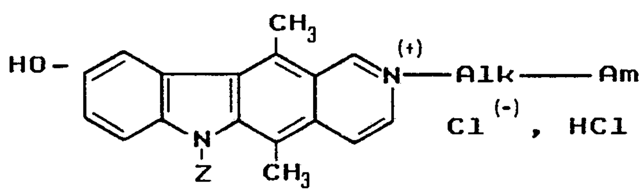

in der bedeuten:

Z Wasserstoff oder niedriges Alkyl, ausgewählt unter Methyl, Ethyl, Propyl, Isopropyl und n-Butyl,
Alk niedriges Alkylen, ausgewählt unter 1,2-Ethylen, 1,2-Propylen, 1,3-Propylen und 1,4-Butylen, und
Am Di-(niedriges Alkyl)-amino, ausgewählt unter Dimethylamino, Diethylamino, Diisopropylamino, Methylethylamino, Methylpropylamino, Ethylpropylamino, Pyrrolidino und Piperidino, dadurch gekennzeichnet, daß
ein 9-Hydroxyellipticin der Formel (III):

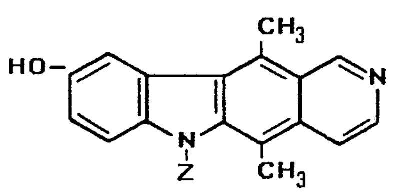

(III)

in der Z wie oben definiert ist, mit einem Chloralkylamin der Formel (IV):

Cl-Alk-Am (IV),

in der Alk und Am wie oben definiert sind,
in einem inerten organischen Lösungsmittel bei einer Temperatur von Raumtemperatur bis 140 °C umgesetzt wird und das so erhaltene quartäre Chlorid der Formel (V):

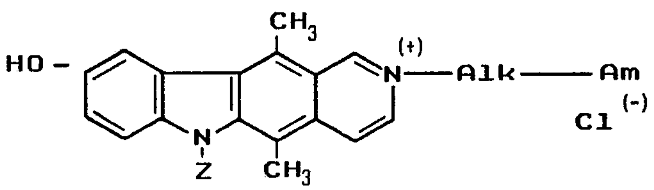

(V)

in der Z, Alk und Am wie oben definiert sind, in das Hydrochlorid durch Behandlung mit Chlorwasserstoffsäure umgewandelt wird.

2. Verfahren nach Anspruch 1, dadurch gekennzeichnet, daß 2-Aminoalkyl-9-hydroxyellipticiniumchlorid-hydrochloride der Formel (II) hergestellt werden, in der Alk Ethylen und Am Diethylamino oder Piperidino ist.

3. Verfahren nach Anspruch 1 zur Herstellung von 2-(2-Diethylaminoethyl)-9-hydroxyellipticiniumchlorid-hydrochlorid.

4. Verfahren nach Anspruch 1 zur Herstellung von 2- (2-Piperidinoethyl)-9-hydroxyellipticiniumchlorid-hydrochlorid.

5. Verfahren zur Herstellung einer pharmazeutischen Zusammensetzung mit Antitumorwirkung, dadurch gekennzeichnet, daß mindestens ein Chlorid-hydrochlorid nach einem der Ansprüche 1 bis 4 mit einem pharmazeutischen Träger kombiniert wird.

**Claims** for the Contracting States: BE, CH, DE, FR, GB, IT, LI, LU NL, SE

1. The hydrochloride of a 2-aminoalkyl-9-hydroxyellipticinium chloride of the formula

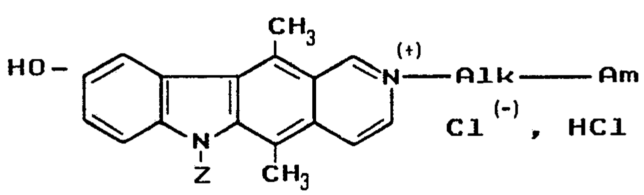

in which Z represents hydrogen or a lower alkyl group selected from methyl, ethyl, propyl, isopropyl and n-butyl groups, Alk represents a lower alkylene group selected from 1,2-ethylene, 1,2-propylene, 1,3-propylene and 1,4-butylene groups and represents a di(lower alkyl)amino group selected from dimethylamino, diethylamino, diisopropylamino, methylethylamino, methylpropylamino, ethylpropylamino, pyrrolidino and piperidino groups.

2. The hydrochloride of a 2-aminoalkyl-9-hydroxyellipticinium chloride according to Claim 1 in which Alk represents an ethylene group and Am represents a diethylamino or piperidino group.

3. The hydrochloride of 2-(2-diethylaminoethyl)-9-hydroxyellipticinium chloride.

4. The hydrochloride of 2-(2-piperidinoethyl)-9-hydroxyellipticinium chloride.

5. The hydrochloride of a 2-aminoalkyl-9-hydroxyellipticinium chloride according to Claim 1 for its application as a therapeutically active substance.

6. The hydrochloride of 2-(2-diethylaminoethyl)-9-hydroxyellipticinium chloride as an antitumoral agent.

7. The hydrochloride of 2-(2-piperidinoethyl)-9-hydroxyellipticinium chloride as an antitumoral agent.

8. A process for the preparation of the hydrochlorides of the chlorides according to Claim 1, characterized in that a 9-hydroxyellipticine of the formula

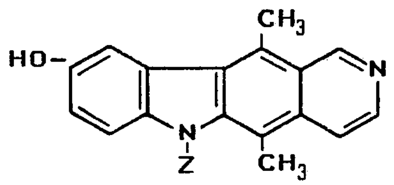

in which Z is as defined above, is treated with a chloroalkylamine of the formula

Cl-Alk-Am

in which Alk and Am are as defined above, in an inert organic solvent at a temperature between room temperature and 140°C, and the resulting quaternary chloride of the formula

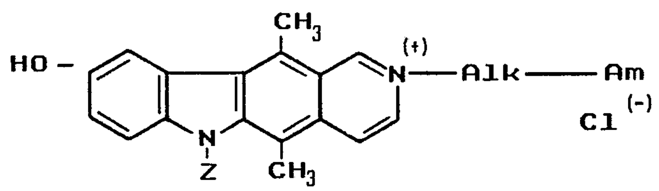

in which Z, Alk and Am are as defined above, is then converted to its hydrochloride by treatment with hydrochloric acid.

9. A pharmaceutical composition, characterized in that it contains at least one hydrochloride of a chloride acoording to any one of Claims 1 to 4.

10. A pharmaceutical composition having an antitumoral action, characterized in that it contains at least one hydrochloride of a chloride according to any one of Claims 1 to 4, in association with a pharmaceutical vehicle.

11. A pharmaceutical composition according to Claim 9 or Claim 10, characterized in that it takes the form of a dosage unit for parenteral administration.

**Claims** for the Contracting State: AT

1. A process for the preparation of the hydrochlorides of the 2-aminoalkyl-9-hydroxyellipticinium chlorides of the formula

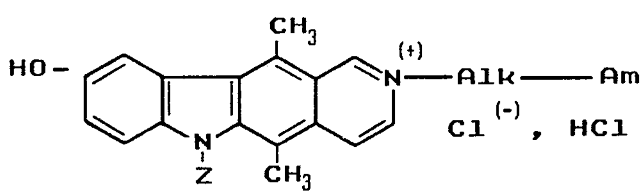

in which Z represents hydrogen or a lower alkyl group selected from methyl, ethyl, propyl, isopropyl and n-butyl groups, Alk represents a lower alkylene group selected from 1,2-ethylene, 1,2-propylene, 1,3-propylene and 1,4-butylene groups and Am represents a di(lower alkyl)amino group selected from dimethylamino, diethylamino, diisopropylamino, methylethylamino, methylpropylamino, ethylpropylamino, pyrrolidino and piperidino groups, characterized in that a 9-hydroxyellipticine of the formula

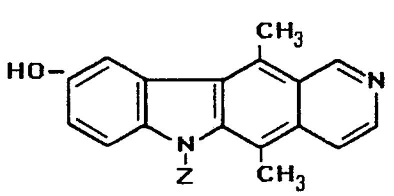

in which Z is as defined above, is treated with a chloroalkylamine of the formula

Cl-Alk-Am

in which Alk and Am are as defined above, in an inert organic solvent at a temperature between room temperature and 140°C, and the resulting quaternary chloride of the formula

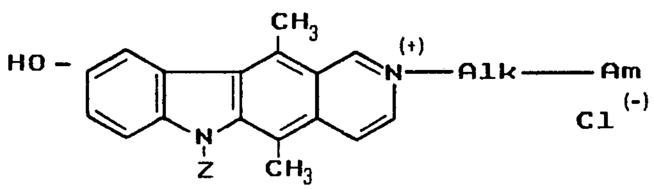

in which Z, Alk and Am are as defined above, is then converted to its hydrochloride by treatment with hydrochloric acid.

2. A process according to Claim 1, characterized in that the hydrochlorides of the 2-aminoalkyl-9-hydroxyellipticinium chlorides in which Alk represents an ethylene group and Am represents a diethylamino or piperidino group are prepared.

3. A process according to Claim 1, characterized in that the hydrochloride of 2-(2-diethylaminoethyl)-9-hydroxyellipticinium chloride is prepared.

4. A process according to Claim 1, characterized in that the hydrochloride of 2-(2-piperidinoethyl)-9-hydroxyellipticinium chloride is prepared.

5. A process for the preparation of a pharmaceutical composition having an antitumoral action, characterized in that at least one hydrochloride of a chloride according to any one of Claims 1 to 4, in association with a pharmaceutical vehicle, is prepared.